# EUROPEAN PATENT SPECIFICATION

(11) **EP 3 795 146 B1**
(45) Date of publication and mention of the grant of the patent: **07.06.2023**
(21) Application number: 20196395.6
(22) Date of filing: 16.09.2020
(51) Int. Cl.: A61K 31/05, A61K 31/618, A61K 31/7008, A61K 31/704, A61K 36/185, A61K 36/324, A61K 38/48, A61P 29/00, A61K 9/00, A61K 9/06, A61K 31/10, A61K 38/07, A61K 47/32, A61K 47/34, A61K 47/44

(54) **PAIN-RELIEVING AND ANTI-INFLAMMATORY COMPOSITION FOR LOCAL USE**
SCHMERZLINDERNDE UND ENTZÜNDUNGSHEMMENDE ZUSAMMENSETZUNG ZUR LOKALEN ANWENDUNG
COMPOSITION ANTI-DOULEUR ET ANTI-INFLAMMATOIRE À USAGE LOCAL

(30) Priority: 19.09.2019 IT 201900016709
(43) Date of publication of application: 24.03.2021
(73) Proprietor: Aqma Italia S.p.A., 20123 Milano (IT)
(72) Inventor: PIRONTI, Michele, 80056 ERCOLANO (IT)
(74) Representative: Coppo, Alessandro

(56) References cited:
- EP-A1- 2 444 081
- WO-A1-2017/059088
- US-A1- 2011 117 207
- US-A1- 2018 092 838
- US-A1- 2019 275 095
- TOZZI E ET AL: "LA FRIGOTERAPIA LOCALE ANTIINFLAMMATORIA CON UNA ASSOCIAZIONE DI ESCINA, EPARINA E FOSFATIDILCOLINA POLINSATURA (EPL)//TOPICAL ANTI INFLAMMATORY THERAPY WITH A COMBINATION OF AESCIN HEPARIN AND POLY UNSATURATED PHOSPHATIDYL CHOLINE", CLINICA TERAPEUTICA, IT, vol. 98, no. 5, 1 January 1981 (1981-01-01), pages 517-524, XP009043462, ISSN: 0009-9074

## Description

### FIELD OF THE INVENTION

The present invention relates to a topical composition for use in the local treatment of inflammation and/or pain of an anatomical structure of the muscle-skeletal system.

The present invention originates in the pharmaceutical sector, in particular in the sector of analgesic-anti-inflammatory products for topical use.

Specifically, the present invention relates to a topical composition for application for the local treatment of pain and acute or chronic inflammatory states of traumatic or rheumatic origin, affecting an anatomical component of the muscle-skeletal system.

### STATE OF THE ART

The wide diffusion of pathologies and disorders affecting the muscle-skeletal system in the population of the most developed countries has contributed to the wide diffusion of medicines provided with antalgic and/or anti-inflammatory action, suitable for oral administration and local application.

Most of these medicines contain non-steroidal anti-inflammatory drugs (NSAIDs), a category of active ingredients with anti-inflammatory and antalgic activity. Notably, NSAIDs act mainly on the metabolism of the arachidonic acid and eicosapentaenoic acid, precursors of substances that are involved in the inflammatory process such as prostaglandins (PG), prostacyclines (PC), thromboxanes (TX) and leukotrienes (LT).

In particular, NSAIDs work by blocking the binding site of cyclooxygenase, enzymes that exist in two isoforms named COX-1 and COX-2.

COX-1 is an enzyme present ubiquitously in the human body under physiological conditions while COX-2 is synthesized inducibly and therefore is present in the body in significant amounts only when an inflammatory process is underway. Numerous formulations, containing NSAIDs as active ingredients having COX-2 as target and that, once administered, reduce the pain associated with inflammatory states of the muscle-skeletal system, have therefore been developed.

From the chemical-pharmaceutical point of view, NSAIDs mainly used in this therapeutic field can be classified, according to the chemical structure of the precursor compound, into:
- derivatives of acetic acid such as indomethacin;
- derivatives of propionic acid such as ibuprofen and ketoprofen;
- phenamate derivatives such as mefenamic acid;
- salicylic derivatives such as acetylsalicylic acid;
- sulfonanilides such as nimesulide;
- oxicams such as piroxicam, meloxicam.

Most of these active ingredients are intended for both oral and parenteral administration. However, the use of topical anti-inflammatory drugs has grown exponentially in recent years. This success is attributable to a number of factors. First of all, the oral administration of NSAIDs, particularly in the chronic treatment of inflammatory states, is associated with the appearance of numerous side effects and an increased risk of developing gastric lesions such as gastro-duodenal ulcers. The appearance of said side effects has limited the systemic use of NSAIDs especially in patients more prone to developing diseases of the gastrointestinal tract.

Another element that has contributed to determining the success of antiinflammatories for local use is connected with the fact that the systemic administration of NSAIDs is also associated with an increased risk of bleeding. These risks and side effects are considerably reduced when NSAIDs are administered locally. In this case, the absorption of the active ingredients and their diffusion in the body is considerably reduced while an appreciable antalgic and anti-inflammatory effect remains at the local level.

These characteristics have made widespread the use of locally applied drugs, which are indicated in the treatment of painful and inflammatory states of traumatic or rheumatic origin of the muscle-skeletal system.

A further incentive to the diffusion of anti-inflammatory drugs for local application is determined by the need to be able to extend to the population of elderly subjects who are more frequently affected by chronic pathologies of the muscle-skeletal system and who at the same time have greater risks of developing side effects associated with the administration of NSAIDs. The need to have anti-inflammatory products readily available in pharmacies and suitable for treating the mildest forms of pain and inflammatory states of the muscle-skeletal system, still remains. Typically, anti-inflammatory drugs for local use are in the form of creams, pomades, gels to be applied to the skin or even medical devices for the prolonged release of the active ingredients in the form of transdermal patches.

However, it has been observed that the formulations of NSAIDs for local use generally cause a lower analgesic and anti-inflammatory response than that obtained with systemic administration.

In view of these advantages, drugs containing NSAIDs for local application however have the drawback of inducing a therapeutic response that is not always adequate in achieving an effective reduction of pain associated with inflammatory states of the muscle-skeletal system structures.

EP 2 444 081 A1 discloses a composition for the treatment of inflammatory diseases, particularly of the skin, mucous membrane or other issues containing 0,01-20wt% of boswellic acids weight in the form of extract or isolate from Boswellia family plants or from the salts of these acids and 0,001 - 30 wt% of cannabidiol. This application also discloses that the composition can be used for treating muscle contractions, muscle pain and strained muscles.

WO 2017/059088 A1 and US 2019/275095 A1 disclose the use of a topical composition comprising CBD hemp oil for treating inflammation associated with musculoskeletal, osteoarthritic, muscle, joint, arthritis, back, strains and sprains pain associated with sport injuries and other disease or trauma.

The publication to Tozzi et al, "La frigoterapia locale antinfiammatoria con una associazione di escina, eparina e fosfatidilcolina polinsatura (EPL)" in Clinica Terapeutica IT vol. 98, n.5, 1 Jan 1981 pages 517-524 , XP009043462 discloses the use of escin for the treatment of joint and muscular diseases.

US 2018/092838 A1 discloses the topical use of MSM for the treatment of osteoarthritis, pain and inflammation.

US 2011/117207 A1 discloses that Boswella serrata, bromelain methylsulfonylmethane (MSM) and glucosamine sulphate are anti-inflammatory and/or joint health promoting compounds.

Currently it is therefore still felt the need to have new remedies, for local use for the treatment of inflammatory states and pain of rheumatic, chronic or traumatic origin, that are alternatives to conventional formulations for topical use based on NSAIDs.

One of the objects of the present invention consists in providing a product and a formulation for topical use alternative to traditional medicines based on NSAIDs, which is provided with antalgic and/or anti-inflammatory properties and which finds application in the treatment of painful states associated with pathologies of traumatic, rheumatic or degenerative origin of structures/annexes that form the muscle-skeletal system.

Another object consists in providing a composition for the treatment of muscle-skeletal pathologies with analgesic and/or anti-inflammatory action whose topical use has a low incidence of side effects.

### SUMMARY

The applicant of the present invention has observed that a specific metabolite of the Cannabis sativa plant has anti-inflammatory and analgesic properties and finds local application in the treatment of muscle-skeletal disorders in the absence of the typical side effects of cannabinoids, when this metabolite is combined with selected active ingredients.

According to one aspect, the combined use of a selected natural cannabinoid present in hemp sativa or in an oily extract from *Cannabis sativa* fruits, in combination with selected active ingredients to locally treat pain or inflammation of a structure of the muscle-skeletal system, is envisaged.

In particular, the applicant has observed that it is possible to use cannabidiol or an oil extracted from hemp Sativa in combination with other selected active components, as an anti-inflammatory and/or local analgesic agent to treat painful and/or inflammatory states and in order to obtain a rapid and permanent reduction of pain, in the orthopedic, rheumatology, trauma field.

Cannabidiol is described herein for local use in the treatment of pain or inflammation of an anatomical structure of the muscle-skeletal system.

Typically for the uses described herein, cannabidiol is dispersed in a pharmaceutically acceptable carrier.

According to one embodiment, cannabidiol is contained in hemp sativa oil. According to a first aspect, the present invention is as defined in claim 1.

Cannabidiol and the compositions containing it described herein find application in the local treatment of painful and inflammatory states, that can affect the joints, like in the case of arthritis and osteoarthritis, the muscles, like in the case of contractures or lesions, tendons and ligaments, like in case of tendonitis.

For example, cannabidiol and the combination compositions containing it described herein find application in the treatment of pain of muscle-skeletal origin which is accompanied by: dislocation of the shoulder, tendonitis of both the lower and upper limbs, sprains of the knee and ankle, nocturnal joint pain, stiff neck and tension states in the neck muscles, neck pain, muscle pains, muscle contractures, torn and pulled muscles, myalgias, leg pain, back pain, lumbago, lumbar pain, low back pain.

Typically, the composition of the invention is in a pharmaceutical form suitable for topical application.

### DETAILED DESCRIPTION OF THE INVENTION

The present invention originates from having identified that cannabidiol, in particular contained in hemp sativa oil, when combined with the selected active ingredients described herein and applied locally on the skin, exerts an action on pain and inflammation affecting an anatomical structure of the muscle-skeletal system, in the absence of the typical side effects of cannabinoids.

According to an aspect of the invention, the topical composition as defined in claim 1 results in a synergistic analgesic, anti-inflammatory effect with a rapid and prolonged reduction of pain associated with muscle-skeletal pathologies. Consequently, the invention is as defined according to claim 1.

Cannabidiol, also known as 2-[(1R,6R)-3-methyl-6-(prop-1-en-2-yl) cyclohex-2-enyl]-5-pentylbenzen-1,3-diol or with the acronym CBD, is a pharmacologically active metabolite of the Cannabis sativa, essentially devoid of psychotropic activity.

As described herein, cannabidiol can be conveyed in a pharmaceutically acceptable carrier substance suitable for topical application.

In accordance with a preferred embodiment, cannabidiol is contained in a hemp sativa oil or extract from hemp sativa.

A suitable extract from hemp sativa containing CDB can be obtained by means of routine techniques.

Preferably, for applications in the medical field of the invention, the hemp oil contains CBD in an amount from 0.05 to 20%, from 0.1 to 10% from 0.5 to 5% by weight.

Typically, hemp sativa oil is extracted from the fruit of Cannabis sativa also known as hemp seed by means of traditional extraction techniques, for example by cold pressing.

By way of example, the unsaponifiable fraction of hemp sativa oil, usable within the scope of the invention, may contain the following substances:

| | |
|---|---|
| Cannabidiol | 10 mg/kg |
| Δ9-Tetrahydrocannabinol | 0-50 ppm |
| Myrcene | 160 ppm |
| β-Caryophyllene | 740 mg/L |
| β-Sitosterol | 100-148 mg/L |
| Campesterol | 25 mg/L |
| α-Tocopherol | 5-80 ppm |
| γ-Tocopherol | 94-870 ppm. |

The Applicant has observed that the local use of Cannabidiol combined with the active components described herein reduces local pain, both in movement and at rest, associated with a muscle-skeletal pathology in a statistically significant manner.

The Applicant has further observed that the reduction of pain and inflammation is achieved in substantial absence of the psychological and neurological side effects that are typical of tetrahydrocannabinol (THC).

In particular, the topical composition for use of the invention, when applied locally, does not lead to the appearance of the following effects: reduction of memory, reduction of thinking and problem-solving ability, reduction of reflexes, alteration of movement coordination, alteration of the sense of time, altered perception of colours or sounds, mood swings, hallucinations and psychosis.

The applicant has also observed that the analgesic/antalgic and anti-inflammatory activities are more marked when cannabidiol is used in combination with escin and/or Boswellia extract.

One of the components of the composition of the invention is escin, a mixture of saponins, substances with anti-inflammatory, vasoprotective and vasoconstrictive activity. Escin is typically extracted from horse chestnut Aesculus hippocastanum and optionally purified by means of traditional techniques.

A further component of the composition of the invention is an extract from Boswellia.

Boswellia is a kind of plant belonging to the Burseraceae's family.

A plant extract of Boswellia serrata, commonly referred to as Boswellia, can be obtained from the roots, leaves, fruits or flowers of the plant.

In some embodiments the plant extract is derived from the stem or trunk or branches of the plant. According to some embodiments, the plant extract of the invention is obtained by extraction from the plant, in particular from its stem or branches or from one of its plant tissues using a pharmaceutically acceptable solvent as the extraction medium. In some embodiments the solvent is hydrophilic and is selected from water, ethanol and mixture thereof.

Methods for obtaining the plant extract of Boswellia serrata include extraction techniques by digestion, infusion, squeezing, decoction, percolation, countercurrent extraction, soxhlet, extraction with supercritical gases or ultrasounds. Typically, the Boswellia extract for the uses of the invention contains gum-resins and oleo-resins, mixtures of triterpenoid resins and essential oils rich in boswellic acids, in particular acetyl-11-keto-beta-boswellic, tetracyclic acids and polysaccharides with anti-inflammatory and analgesic action, with a chemical structure similar to that of anti-inflammatory steroids.

The combination of Boswellia and Cannabidiol reduces the synthesis of leukotrienes, substances involved in arthritic and rheumatic inflammatory processes, limiting the destructive action of elastases, the enzymes responsible for demolishing inflamed tissues.

The composition for use of the invention further contains Coco-Glucoside and Acetyl Tetrapeptide-15, typically conveyed in water and glycerin. A product with these characteristics is marketed under the brand Skinasensyl LS 9749 by Basf. The mixture of Coco-Glucoside and Acetyl Tetrapeptide-15 is the basic component of the Skinasensyl LS9749^{®} product. In this product Coco-Glucoside and Acetyl Tetrapeptide-15 are dispersed in water and glycerin.

The term Tetrapeptide-15 means the peptide: N-acetyl-L-tyrosyl-L-prolyl-L-phenylalanyl-L-phenylalaninamide (IUPAC).

Coco-Glucoside is a non-ionic surfactant. Chemically, it is an ether from C8-16 fatty alcohols and glucose oligomers. It comes in the form of a viscous, cloudy aqueous solution. Typically, the percentage of active substance ranges generally from 50 to 60% by weight.

The composition for use of the invention further contain one or more active components selected from bromelain, glucosamine sulfate, methylsalicylate, methylsulfonylmethane and a mixture of Coco-Glucoside and Acetyl Tetrapeptide-15.

Each of these components of the composition further contributes to producing the analgesic and anti-inflammatory activity.

Bromelain comprises two proteolytic enzymes identified in Ananas comosus and has an anti-inflammatory and anti-oedematous action.

Methyl sulfonylmethane or dimethylsulfone is a compound with anti-inflammatory and chondroprotective activity.

Glucosamine sulfate or 2-D-(+)-glucosamine is an aminomonosaccharide with analgesic and anti-inflammatory properties.

Methylsalicylate is the ester of salicylic acid and methanol and is a molecule with analgesic properties that finds application also as a local anesthetic.

Each pharmacologically active component of the formulation is present in the composition in an amount ranging from 0.01 to 30%, from 0.1 to 15%, from 0.5 to 10% by weight.

In this context, the terms active component, pharmacologically active component, pharmacologically active ingredient are to be considered substantially equivalent. The composition for use of the invention can be used for the same therapeutic indications as cannabidiol or hemp sativa oil previously described.

Consequently, the composition according to any one of the embodiments described herein finds application in the local treatment of inflammatory and painful states of traumatic, chronic, or rheumatic origin of the joints, muscles, tendons and ligaments.

In some embodiments the composition for use of the invention comprises a pharmaceutically acceptable carrier, diluent, or excipient.

Typically, the pharmaceutically acceptable carrier of the composition of the invention is an excipient, vehicle, or diluent suitable for the application.

In the context of the present invention, the term "carrier" refers to an excipient, vehicle, diluent, or adjuvant which may be present in the composition of the invention.

The administration route of the composition for use of the invention is the topical administration route.

The composition for use of the invention can therefore be applied, in a therapeutically effective amount, directly on the skin in the area affected by pain or inflammation of an anatomical structure of the muscle-skeletal system.

The topical composition for topical application as herein disclosed can be in solid, semi-solid or fluid form.

Suitable formulations in solid form not part of the present invention include creams, pomades, pastes, ointments.

In other embodiments, the formulation for local administration is in fluid or semi-solid form, for example in the form of gels, suspensions, emulsions, oils.

The topical composition for use according to the invention are in the form of a gel or lipogel or an emulsion comprising a mixture of Coco-Glucoside and acetyl tetrapeptide-15.

By way of example, a suitable lipogel is prepared by dispersing silica in the liquid phase. The gels that are formed thanks to hydrogen bonds that are established between the silanol residues have a thixotropic/pseudoplastic behaviour, i.e. their viscosity increases at rest and is reduced after stirring or mixing. Suitable types of hydrophilic silica are aerosil 200^{®} and aerosil 300^{®} which provide viscous and stable gels. Lipogels are emollient and protective indicated in barrier preparations and in those intended for sensitive and fragile skin such as in diabetics, the elderly and children.

In one embodiment, the composition as herein disclosed contains hemp sativa oil conveyed within the lipid component of liposomes or phospholipid micelles to increase absorption of the active CBD component through the skin. Suitable liposomes are vesicles or microspheres based on mono- or multi-layered phospholipids. In these embodiments, the phospholipids used as carriers comprise a hydrophobic tail based on fatty acids and a hydrophilic head comprising a phosphate group bound to a polar group, for example choline, serine, ethanolamine. In accordance with one embodiment, the phospholipids comprise phosphatidylcholine.

In the case of formulations in fluid or semi-fluid form, the active components can be diluted in a carrier in a physiologically acceptable liquid form such as water, alcohol, hydroalcoholic or glycerine solution or mixed with other liquids suitable for local application.

In some embodiments the compositions for the use of the invention may comprise excipients commonly used in the formulation of pharmaceutical preparations, for local use, such as preservatives, bactericidal agents, stabilizers, emulsifiers, buffers, humectants, dyes and other excipients commonly used in pharmaceutical preparation techniques.

Typically, the composition for use of the invention contains suitable carriers in liquid form such as for example water, alcohol, for example ethyl alcohol, glycols such as propylene glycol, caprylyl glycol, decylene glycol, glycerin Examples of suitable excipients possibly present in the composition include Maltodextrin, carbomer, phenoxyethanol, sodium dihydroacetate, polyvinylpyrrolidone, lubricants such as triacylglycerols, hydroxymethylcellulose, gelling agents with HLB and mixtures thereof. The composition for use according to the invention contains an emollient agent such as Coco-Glucoside and Acetyl Tetrapeptide-15.

The composition for the use of the invention finds application in the orthopedic, rheumatological and traumatological fields, in particular in the treatment of pains and inflammatory states.

In particular, the composition for use of the invention is indicated in the local treatment of painful and/or inflammatory states affecting one or more anatomical structures such as:
- joints, for example in the treatment of osteoarthrosis, arthritis,
- muscles, for example in the treatment of contractures, strain or lesions,
- tendons or ligaments, for example in the treatment of tendonitis.

For example, the composition for the use of the invention and/or a gel containing it finds application in the treatment of pain and/or inflammation associated with dislocation of the shoulder, tendonitis, sprains of the knee and ankle, nocturnal joint pains, muscle pains, myalgia, muscle contractures, torn and pulled muscles, muscle strains, myalgias, leg pain, back pain, lumbago, lumbar pain, low back pain.

The composition for the use of the invention provides for the application thereof on the skin of the area affected by pain or inflammation of the anatomical structure of interest. The skin application of the composition can be repeated for example 1-4 times a day. For example, an amount of composition ranging from 1 to 5 g is applied locally in a 300-900 cm² area of the body in need of treatment.

The present invention is described with reference to the following examples.

### EXAMPLES

### Example 1:

Topical composition in the form of gel and/or emulgel and/or lipogel and/or oil-in-water emulsion for the use in the local treatment of inflammation and/or pain having the following formulation:

| Active ingredient | Amount by weight% |
|---|---|
| Hemp oil with CBD | 0.1 |
| Escin | 2 |
| Boswellia extract | 10 |
| Skinasensyl Is | 2 |
| Bromelain | 1 |
| Methylsulfonylmethane | 1 |
| Glucosamine sulfate | 1 |
| Methylsalicylate | 0,1 |
| Excipients (Water, ethyl alcohol, carbopol, triethanolamine) | q.s. at 100 |

### Example 2

Topical composition in the form of gel and/or emulgel and/or lipogel and/or oil-in-water emulsion for the use in the local treatment of muscle pains or inflammatory states of the joints having the following formulation:

| Active ingredient | Amount by weight% |
|---|---|
| Hemp oil containing CBD | 0.2 |
| Escin | 3 |
| Boswellia extract | 10 |
| Skinasensyl Is | 4 |
| Bromelain | 3 |
| Methylsulfonylmethane | 5 |
| Glucosamine sulfate | 2 |
| Methylsalicylate | 0,3 |
| Excipients: | q.s. at 100 |

Water, isopropyl alcohol, diethylamine, carbopol, macrogol, liquid paraffin, cetostearyl ether, cocoyl caprylocaprate, propylene glycol.

## Claims

1. A topical composition for use in the local treatment of inflammation and/or pain of an anatomical structure of the muscle-skeletal system, comprising cannabidiol or an extract from hemp sativa containing it in combination with escin, a Boswellia extract, bromelain, glucosamine sulfate, methylsalicylate, methylsulfonylmethane and a pharmaceutically acceptable carrier, wherein the composition is in the form of a gel, lipogel or an emulsion comprising a mixture of Coco-Glucoside and Acetyl Tetrapeptide-15.

2. The topical composition for use according to Claim 1, wherein the mixture of Coco-Glucoside and Acetyl Tetrapeptide-15 is dispersed in a water-based liquid and glycerine.

3. The topical composition for use according to anyone of Claims 1-2, wherein the cannabidiol is contained in hemp sativa oil.

4. The topical composition for use according to anyone of Claims 1-3, wherein the cannabidiol is contained in an amount comprised in the range from 0.1 to 10% by weight.

5. Topical composition in the form of a gel or lipogel for use according to anyone of claims 1-4 having the following composition by weight
| | |
|---|---|
| Hemp oil containing CBD | 0.1% |
| Escin | 2% |
| Boswellia extract | 10% |
| Skinasensyl Is | 2% |
| Bromelain | 1% |
| Methylsulfonylmethane | 1% |
| Glucosamine sulfate | 1% |
| Methylsalicylate | 0.1% |
| Water, ethyl alcohol, carbopol, triethanolamine | q.s. at 100% |

6. The topical composition for use according to anyone of Claims 1-5 in the local treatment of inflammatory and painful states of traumatic, chronic or rheumatic origin of the joints, muscles, tendons and/or ligaments.

7. The topical composition for use according to anyone of Claims 1-6 in the local treatment of a muscle-skeletal disease or disorder of the human body.

## Patentansprüche

1. Topische Zusammensetzung zur Verwendung bei der lokalen Behandlung von Entzündungen und/oder Schmerzen einer anatomischen Struktur des Muskel-Skelett-Systems, umfassend Cannabidiol oder einen es enthaltenden Extrakt aus Cannabis sativa in Kombination mit Escin, einem Boswellia-Extrakt, Bromelain, Glucosaminsulfat, Methylsalicylat, Methylsulfonylmethan und einen pharmazeutisch annehmbaren Träger, wobei die Zusammensetzung in Form eines Gels, Lipogels oder einer Emulsion vorliegt, die eine Mischung aus Coco-Glucosid und Acetyl-Tetrapeptid-15 umfasst.

2. Topische Zusammensetzung zur Verwendung nach Anspruch 1, wobei die Mischung aus Coco-Glucosid und Acetyl-Tetrapeptid-15 in einer Flüssigkeit auf Wasserbasis und Glycerin dispergiert ist.

3. Topische Zusammensetzung zur Verwendung nach einem der Ansprüche 1-2, wobei das Cannabidiol in Cannabis-Sativa-Öl enthalten ist.

4. Topische Zusammensetzung zur Verwendung nach einem der Ansprüche 1-3, wobei das Cannabidiol in einer Menge im Bereich von 0,1 bis 10 Gewichtsprozent enthalten ist.

5. Topische Zusammensetzung in Form eines Gels oder Lipogels zur Verwendung nach einem der Ansprüche 1-4 mit der folgenden Gewichtszusammensetzung
| | |
|---|---|
| CBD-haltiges Hanföl | 0,1 % |
| Escin | 2 % |
| Boswellia-Extrakt | 10 % |
| Skinasensyl Is | 2 % |
| Bromelain | 1 % |
| Methylsulfonylmethan | 1 % |
| Glucosamin-Sulfat | 1 % |
| Methylsalicylat | 0,1 % |
| Wasser, Ethylalkohol, Carbopol, Triethanolamin | q.s. bei 100% |

6. Topische Zusammensetzung zur Verwendung nach einem der Ansprüche 1-5 bei der lokalen Behandlung von entzündlichen und schmerzhaften Zuständen traumatischen, chronischen oder rheumatischen Ursprungs der Gelenke, Muskeln, Sehnen und/oder Bänder.

7. Topische Zusammensetzung zur Verwendung nach einem der Ansprüche 1-6 bei der lokalen Behandlung einer Muskel-Skelett-Erkrankung oder -Störung des menschlichen Körpers.

## Revendications

1. Composition topique destinée à être utilisée dans le traitement local de l'inflammation et/ou de la douleur d'une structure anatomique du système musculo-squelettique, comprenant du cannabidiol ou un extrait de chanvre le contenant en association avec de l'escine, un extrait de Boswellia, de la bromélaïne, du sulfate de glucosamine, du méthylsalicylate, du méthylsulfonylméthane et un support pharmaceutiquement acceptable, dans laquelle la composition est sous la forme d'un gel, d'un lipogel ou d'une émulsion comprenant un mélange de coco-glucoside et d'acétyle tétrapeptide-15.

2. Composition topique destinée à être utilisée selon la revendication 1, dans laquelle le mélange de coco-glucoside et d'acétyle tétrapeptide-15 est dispersé dans un liquide à base d'eau et de glycérine.

3. Composition topique destinée à être utilisée selon l'une quelconque des revendications 1 à 2, dans laquelle le cannabidiol est contenu dans l'huile de chanvre.

4. Composition topique destinée à être utilisée selon l'une quelconque des revendications 1 à 3, dans laquelle le cannabidiol est contenu en une quantité comprise dans une plage de 0,1 à 10 % en poids.

5. Composition topique sous forme d'un gel ou d'un lipogel destinée à être utilisée selon l'une quelconque des revendications 1 à 4, ayant la composition suivante en poids
| | |
|---|---|
| Huile de chanvre contenant du CBD | 0,1 % |
| Escine | 2 % |
| Extrait de Boswellia | 10 % |
| Skinasensyl LS | 2 % |
| Bromélaïne | 1 % |
| Méthylsulfonylméthane | 1 % |
| Sulfate de glucosamine | 1 % |
| Méthylsalicylate | 0,1 % |
| Eau, alcool éthylique, carbopol, triéthanolamine | q.s. à 100 % |

6. Composition topique destinée à être utilisée selon l'une quelconque des revendications 1 à 5 dans le traitement local d'états inflammatoires et douloureux d'origine traumatique, chronique ou rhumatismale des articulations, des muscles, des tendons et/ou des ligaments.

7. Composition topique destinée à être utilisée selon l'une quelconque des revendications 1 à 6 dans le traitement local d'une maladie ou d'un trouble musculo-squelettique du corps humain.
